# FASCICULE DE BREVET EUROPEEN

(11) **EP 0 721 920 B1**
(45) Date de publication et mention de la délivrance du brevet: **26.09.2001**
(21) Numéro de dépôt: 96460001.9
(22) Date de dépôt: 12.01.1996
(51) Int. Cl.: C02F 1/32

(54) **Réacteur d'irradiation uv pour le traitement de liquides**
UV-Bestrahlungsreaktor zur Flüssigkeitenreinigung
UV irradiation reactor for the treatment of liquids

(30) Priorité: 16.01.1995 FR 9500616
(43) Date de publication de la demande: 17.07.1996
(73) Titulaire: OTV Omnium de Traitements et de Valorisation S.A., 94417 St Maurice Cédex (FR)
(72) Inventeur: Binot, Patrick, F-77400 Bussy Saint-Martin (FR)
(74) Mandataire: Vidon, Patrice

(56) Documents cités:
- EP-A- 0 470 518
- WO-A-93/02965
- DE-A- 3 414 870
- DE-A- 3 924 349
- US-A- 4 273 660
- US-A- 5 124 131

## Description

La présente invention est relative à la désinfection des liquides, notamment l'eau potable ou les eaux usées, contaminées par des micro-organismes, à l'aide de lampes UV haute ou moyenne pression.

Plus particulièrement, l'invention porte sur diverses dispositions originales permettant d'améliorer l'efficacité de la désinfection UV haute ou moyenne pression.

Les micro-organismes, soumis à un bombardement de photons UV, subissent des modifications de leur ADN pouvant en interdire la réplication. La proportion statistique de micro-organismes d'un type donné ainsi inactivée au sein d'une population donnée est proportionnelle à la dose UV (produit de l'intensité de rayonnement par le temps d'exposition) reçue par la population considérée. Certaines longueurs d'onde, en particulier la longueur d'onde égale à 254 nm, ont une meilleure efficacité germicide que les autres.

Deux types principaux d'émetteurs de rayonnement UV sont actuellement disponibles dans le commerce.

Les lampes UV à basse pression de vapeur de mercure (lampes UV - BP) qui ont l'avantage d'émettre un rayonnement quasi monochromatique, sur 254 nm, donc à la longueur d'onde d'efficacité germicide optimale, mais qui présentent l'inconvénient d'être limitées en puissance unitaire. Les lampes UV - BP du commerce sont actuellement disponibles dans la gamme 50 - 200 watts environ. Un grand nombre de lampes doit donc généralement être installé pour traiter un débit d'eau usée moyen.

Les lampes UV à haute ou moyenne pression de vapeur de mercure (lampes UV-HP ou UV - MP), ont un spectre d'émission qui est plus étalé et sont donc de moindre efficacité germicide, mais présentent une puissance unitaire pouvant être très élevée (des lampes de 3 kw, 10 kw et jusqu'à 100 kw sont actuellement disponibles).On notera que l'on désigne dans le cadre de la présente description sous les termes émetteurs UV haute ou moyenne pression, les émetteurs UV d'une puissance d'au moins 500 w, classiquement de 3 à 100 kw.

Essentiellement deux types de réacteurs UV sont utilisés dans l'industrie de traitement de l'eau.

D'une part les réacteurs de type ouvert, ou en canal, dans lequel le liquide en écoulement gravitaire à surface libre passe au milieu d'une multitude de lampes équiréparties parallèlement entre elles dans le canal (en position verticale, horizontale ou inclinée).

D'autres part les réacteurs de type fermé, généralement sous pression, dans lesquels l'eau à traiter circule dans une chambre contenant un ou plusieurs émetteurs UV parallèles à l'axe de la chambre et répartis suivant un pas constant dans une section donnée de la chambre. De tels réacteurs de type fermé peuvent notamment être constitués par un émetteur UV disposé coaxialement à l'intérieur d'une gaine de protection transparente aux UV, l'ensemble constitué par ledit émetteur et ladite gaine étant lui-même installé coaxialement à l'intérieur d'une virole tubulaire, le liquide à traiter transitant selon un écoulement tubulaire dans une chambre active délimitée par la face intérieure de ladite virole et la face extérieure de ladite gaine. C'est plus précisément à ce type de réacteur fermé que se rapporte l'invention.

De tels réacteurs sont notamment décrits dans US-A-4,273,660, EP-A-3 924 349 ou encore WO 93/02965.

On notera que des réacteurs de ce type pourront comprendre plusieurs émetteurs et plusieurs chambres actives, chaque émetteur étant disposé coaxialement dans une gaine de protection transparente aux UV et chaque émetteur desservant une seule chambre active.

L'utilisation des lampes UV - HP ou MP a tendance à s'imposer dans le domaine du traitement des eaux potables, dans la mesure où la compacité, la simplicité, et l'économie sur investissement permises par l'utilisation de lampes UV - HP en réacteurs fermés fait plus que compenser l'augmentation de la consommation électrique due à la moindre efficacité germicide des lampes UV - HP ou UV - MP par rapport aux lampes UV - BP.

Dans le domaine des eaux usées, la technologie la plus utilisée actuellement reste la technologie UV - BP en réacteurs ouverts, comme décrite notamment dans les demandes de brevet européen EP-A-361 579A1 et EP-A-80 780. En effet les eaux usées traitées présentant couramment des coefficients de transmissions faibles (40 % à 60 % sur lame de 1 cm) donnant ainsi à la consommation électrique une plus grande importance qu'en eau potable. Les lampes UV- BP, qui présentent une meilleure efficacité germicide du rayonnement produit, sont donc actuellement souvent préférées en traitement des eaux usées.

Les lampes UV - HP ou MP présentent cependant de nombreux avantages potentiels qui pourraient les rendre préférables aux lampes UV - BP :
- meilleure adaptation à la régulation automatique de la puissance rayonnée aux besoins (les lampes BP fonctionnent en tout ou rien, les lampes HP/ MP peuvent être régulées par palier de puissance ou en continu) ;
- meilleure adaptation à un nettoyage automatisé, les lampes UV - BP nécessitant dans la pratique des lavages manuels périodiques consommateurs de main d'oeuvre et générateurs potentiels de casse de lampes et de gaines de quartz ;
- meilleure maîtrise de l'hydraulique du fluide à traiter possible dans le cas des lampes UV - HP (MP) uniques, coaxiales au réacteur, par comparaison à l'hydraulique observée dans les canaux multilampes. La maîtrise hydraulique est en effet essentielle pour assurer une dose de radiation statistiquement identique pour toute particule entraînée par le fluide traité.

Toutefois, les réacteurs UV- HP (ou MP) à lampe coaxiale disponibles dans le commerce et décrits dans la littérature ne permettent pas de minimiser la perte énergétique liée à l'encrassement des éléments émetteurs UV.

Jusqu'ici, la minimisation liée à l'encrassement n'a pu être obtenue qu' à l'aide d'un film plastique protecteur, par exemple en appliquant une fine couche de fluoroéthylène propylène (FEP), comme proposé par le brevet WO 91/09673 du 11.07.91 ou par le brevet WO 88/04281 du 9.12.87, sur cette gaine, ou par l'utilisation de mécanismes racleurs, plus ou moins compliqués et susceptibles de pannes, pour nettoyer périodiquement la surface du tube transparent aux UV protégeant l'émetteur UV, tel celui proposé par le brevet WO92/22502. Ces films et racleurs sont usuellement peu efficaces contre les dépôts minéraux tels que le carbonate de calcium et ne sont donc pas adaptés au traitement des liquides relativement chargés en impuretés tels que notamment les eaux usées.

Par ailleurs, les réacteurs de type fermé ne permettent également pas d'uniformiser le temps de séjour dans le réacteur en limitant efficacement et à bas coût en équipement, les courts circuits hydrauliques importants induits par l'entrée et par la sortie désaxées du liquide à traiter dans le réacteur.

L'objectif principal de la présente invention est d'améliorer l'efficacité des réacteurs à lampe UV - HP ou UV - MP coaxiale de façon à les rendre économiquement intéressants pour le traitement de tels liquides, en retardant au maximum l'encrassement de la chambre active et de la gaine protégeant l'émetteur UV d'un tel réacteur en permettant leur désencrassement automatique lorsque cela est nécessaire.

Un autre objectif de l'invention est d'améliorer l'efficacité germicide des dispositifs de désinfection UV - HP ou MP utilisant une lampe coaxiale à un tube en optimisant la dose d'irradiation de chaque particule du liquide traité dans un tel réacteur et en améliorant la turbulence de ce liquide.

Jusqu'à présent les techniques utilisées pour procurer la turbulence du liquide dans le réacteur ont consisté à homogénéiser les doses de liquide appliquées par mélange radial, par interposition de dispositifs divers du type diaphragme, chicane, etc...comme il est notamment décrit dans la demande de brevet EP-A-202 820.

Ces différents objectifs, ainsi que d'autres qui apparaîtront par la suite, sont atteints grâce à l'invention qui concerne un réacteur d'irradiation UV, de type fermé, pour la désinfection de liquides comportant au moins un émetteur UV haute ou moyenne pression disposé coaxialement à l'intérieur d'une gaine de protection transparente aux UV, l'ensemble constitué par ledit émetteur et ladite gaine étant installé coaxialement à l'intérieur d'une virole tubulaire dont la face intérieure réfléchit au moins 30 % des UV 250 à 260 nm incidents provenant dudit émetteur, le liquide à traiter transitant selon un écoulement tubulaire dans une chambre active délimitée par ladite face intérieure de ladite virole et la face extérieure de ladite gaine, ledit réacteur étant caractérisé en ce qu'il est relié à des moyens de lavage de ladite chambre active incluant des moyens permettant de faire circuler un liquide de nettoyage dans ladite chambre active et à des moyens d'injection d'air prévus en amont de ladite chambre active permettant notamment d'améliorer la turbulence dudit liquide de nettoyage lors du lavage,
en ce qu'il présente des moyens permettant de faire subir au liquide à traiter entrant dans ladite chambre active une perte de charge symétrique au moins trois fois supérieure à la perte de charge dissymétrique observée par le liquide à traiter avant son entrée dans ledit réacteur,
et en ce qu'il comprend une chambre d'injection prévue coaxiale à une extrémité de ladite virole et au moins une tubulure permettant l'introduction du liquide à traiter dans ledit réacteur via ladite chambre d'injection ladite chambre d'injection communiquant avec ladite chambre active par une fente circulaire.

L'utilisation d'un liquide de nettoyage dans les réacteurs en canal était inconcevable de par la configuration ouverte même de ceux-ci. En ce qui concerne les réacteurs de type fermé, on connaissait l'utilisation des râcleurs et/ou de liquides de nettoyage.

Selon l'invention, ledit réacteur inclut également des moyens d'injection d'air permettant d'améliorer la turbulence du liquide de nettoyage lors du lavage. L'air ainsi utilisé permet "d'assister" le liquide de nettoyage sans posséder en lui-même d'effet nettoyant marqué.

On notera que l'utilisation d'une injection d'air n'avait également jamais été envisagée dans le cadre de réacteur de type fermé dans le but d'améliorer l'efficacité d'un liquide de nettoyage. Seul le document US-A-4,273,660 préconisait l'injection d'air ayant transité dans l'espace prévu entre l'émetteur et la gaine de protection, et présentant ainsi une certaine quantité d'ozone, ayant pour seul objet d'accentuer la désinfection de l'eau transitant dans la chambre active.

Le réacteur décrit dans ce document US-A-4273660 inclut une chambre active dans laquelle le liquide à traiter additionné d'air est introduit par une buse. Une telle buse est prévue pour provoquer un fort cisaillement des bulles de gaz. Cette buse présente ainsi l'inconvénient d'augmenter fortement la perte de charge dissymétrique du fluide à l'entrée de la chambre active, ce qui crée un jet dans celle-ci source d'une mauvaise répartition des temps de séjour des particules d'eau à traiter dans la zone de traitement. La présente invention permet au contraire, grâce aux moyens permettant de faire subir au liquide à traiter entrant dans ladite chambre active une perte de charge symétrique au moins trois fois supérieure à la perte de charge dissymétrique observée par le liquide à traiter avant son entrée dans ledit réacteur, de garantir une distribution homogène du fluide à traiter dans la chambre active du réacteur et donc une bonne homogénéité des temps de séjour de chaque particule entrante. Ceci permet d'optimiser le fonctionnement du réacteur.

Les moyens de lavage pourront être mis en oeuvre selon une grille horaire mais préférentiellement, le réacteur selon l'invention inclura des moyens d'activation automatique des moyens de lavage. Ces moyens d'activation automatique pourront préférentiellement comprendre un capteur UV prévu au niveau de la virole du réacteur et permettant d'évaluer la transmission UV de la lame de liquide présente dans la chambre active du réacteur et le taux d'encrassement de cette chambre.

Egalement préférentiellement, lesdits moyens de lavage incluent des moyens permettant de recycler ledit liquide de nettoyage.

Egalement avantageusement, ces moyens de lavage comprennent aussi des moyens de réajustement du titre de celui-ci.

Préférentiellement, le réacteur inclut des moyens d'activation automatique desdits moyens de lavage.

Egalement préférentiellement, lesdits moyens d'activation comprennent un capteur UV prévu au niveau de ladite virole du réacteur.

Avantageusement, le réacteur comprend des moyens de chauffage dudit liquide de nettoyage.

Comme précisé ci-dessus, la face intérieure de la virole réfléchit au moins 30 % des UV 250 à 360 nm incidents provenant de l'émetteur.

Typiquement, selon les matériaux utilisés pour constituer la paroi intérieure de cette virole une telle réflexion sera comprise entre 35 et 60 %

L'énergie récupérée grâce à une telle caractéristique est utilisée pour la désinfection des parties de l'écoulement les plus éloignées de l'émetteur UV, c'est-à-dire les parties de l'écoulement les moins irradiées. L'invention autorise donc un complément d'irradiation qui permet d'améliorer la performance globale du traitement d'irradiation.

Selon une variante préférentielle de l'invention, ladite virole est en acier inoxydable, de préférence 304L ou 316L et sa face intérieure présente un poli miroir de grain 100 à 350. De façon imprévue il a en effet été constaté que de tels aciers inoxydables ainsi traités permettait l'optimisation énergétique du réacteur en cumulant les caractéristiques suivantes :
- une meilleure résistance à l'encrassement par les matières organiques contenues dans le liquide à traiter (et une plus grande faculté à être nettoyé...), permettant de maintenir plus longtemps l'efficacité de la désinfection ;
- un coefficient de réflexion de la surface propre k environ égal à 0.40 et approximativement double de celui obtenu sur la tôle inox non polie, permettant une récupération de plus de 40 % de l'énergie UV incidente (aux longueurs d'onde d'environ 250 à 260 nm).

L'utilisation d'une virole en acier inoxydable dont la paroi intérieure présente un poli miroir de grain supérieur à 100 et l'utilisation de moyens d'injection d'un liquide de lavage assisté a l'air concourrent donc par synergie à une meilleure efficacité du réacteur selon l'invention.

On notera que d'autres métaux que l'acier inoxydable donnent également de bons résultats de réflexion, tels que l'aluminium, qui a toutefois une plus forte tendance à la corrosion par les liquides traités, ou le chrome.

Selon une autre variante de l'invention, ladite face intérieure de ladite virole pourra aussi être constituée par une couche métallique obtenue par dépôt électrolytique.

Par ailleurs, avantageusement, le réacteur selon l'invention est dimensionné de façon à inculquer, au débit considéré de liquide à traiter, un nombre de Reynolds supérieur à 6000 dans l'écoulement tubulaire interne du réacteur. La dose reçue par une particule transitant dans le réacteur à une distance r de l'émetteur est essentiellement fonction de cette distance r d'après la loi de Beer-Lambert. Selon l'invention les dimensions du réacteur sont donc prévues pour que chaque élément du fluide à traiter reçoive une dose similaire d'irradiation et permette d'assurer un écoulement turbulent de nombre de Reynolds supérieur à 6000. Typiquement le nombre de Reynolds du fluide en écoulement sera ainsi compris entre 10 000 et 60 000.

Avantageusement, le réacteur comprend des moyens d'injection d'au moins un réactif apte à compléter la désinfection et/ou à détoxifier ledit liquide, lesdits moyens d'injection étant prévus en amont de ladite chambre active.Ces moyens d'injection d'au moins un réactif (ozone, eau oxygénée ...) pourront être constitués par les moyens d'injection d'air cités ci-dessus.

Avantageusement, ledit réacteur comprend des moyens de régulation de la puissance dudit émetteur en fonction d'un ou plusieurs paramètres choisis dans le groupe constitué par le débit de liquide traité, le transmission UV du liquide traité, la durée écoulée depuis le dernier lavage.

Lorsque le réacteur sera destiné à être placé dans une chambre de plus grandes dimensions limitant l'effet de l'arrivée dissymétrique du liquide à traiter, il ne présentera pas de chambre d'injection mais, selon un autre aspect de l'invention, un espace annulaire compris entre ladite virole tubulaire et ladite gaine de protection et séparé de ladite chambre active par une passage rétréci.

La présente invention couvre donc également un réacteur d'irradiation UV pour la désinfection de liquides comportant au moins un émetteur UV haute ou moyenne pression disposé coaxialement à l'intérieur d'une gaine de protection transparente aux UV, l'ensemble constitué par ledit émetteur et ladite gaine étant installé coaxialement à l'intérieur d'une virole tubulaire dont la face intérieure de ladite virole réfléchit au moins 30 % des UV 250 à 260 nm incidents provenant dudit émetteur, le liquide à traiter transitant selon un écoulement tubulaire dans une chambre active délimitée par ladite face intérieure de ladite virole et la face extérieure de ladite gaine, ledit réacteur étant destiné à être placé dans une cuve de plus grandes dimensions limitant l'effet de l'arrivée dissymétrique du liquide à traiter
caractérisé en ce qu'il est relié à des moyens de lavage de ladite chambre active incluant des moyens permettant de faire circuler un liquide de nettoyage dans ladite chambre active et des moyens d'injection d'air prévus en amont de ladite chambre active permettant d'améliorer la turbulence dudit liquide de nettoyage lors du lavage,
en ce qu'il présente des moyens permettant de faire subir au liquide à traiter entrant dans ladite chambre active une perte de charge symétrique au moins trois fois supérieure à la perte de charge dissymétrique observée par le liquide à traiter avant son entrée dans ledit réacteur,
en ce qu'il présente dans sa partie inférieure un espace annulaire compris entre ladite virole tubulaire et ladite gaine de protection et séparé de ladite chambre active par une passage rétréci.

L'invention concerne également un procédé d'utilisation d'un réacteur selon l'invention caractérisé en ce qu'il consiste à faire fonctionner ledit émetteur lorsque les moyens de lavage sont mis en oeuvre. Un tel procédé permet de prolonger la durée de vie de l'émetteur.

De façon curieuse et inattendue, il a été par ailleurs constaté que l'efficacité germicide d'une unité énergétique consommée par un émetteur UV HP/MP était d'autant meilleure, exprimée en terme d'abattement logarithmique des germes, que l'émetteur était utilisé à un niveau de puissance faible par rapport à sa puissance nominale.

C'est ainsi que si 10 émetteurs 10 kw unitaires permettent de traiter , par exemple, un débit de pointe de 1000 m3/h d'une eau usée donnée, il sera plus efficace de traiter un débit moyen de 600 m3/h avec 10 lampes fonctionnant à 6 kw unitaire qu'avec 6 émetteurs à leur puissance nominale de 10 kw.

L'invention couvre donc également un procédé de régulation d'un système de plusieurs émetteurs UV HP/MP selon lequel la puissance nécessaire au traitement d'un débit global donné est obtenue en répartissant uniformément la puissance totale nécessaire sur le nombre d'émetteurs disponibles, plutôt qu'en utilisant quelques émetteurs seulement à leur puissance maximale.

Bien entendu ce procédé ne sera appliqué, en cas de débits de plus en plus faible, que jusqu'à la puissance unitaire minimale acceptable pour chaque émetteur.

L'invention, ainsi que les différents avantages qu'elle présente, seront plus facilement compris grâce à la description qui va suivre d'exemples non limitatifs de réalisation de celle-ci en référence aux dessins dans lesquels :
- la figure 1 montre une vue latérale en coupe longitudinale d'un premier mode de réalisation de l'invention ;
- la figure 2 montre une vue en coupe transversale AA du réacteur représenté à la figure 1;
- la figure 3 montre une vue schématique en coupe du réacteur selon les figures 1 et 2 et des moyens de lavage de la chambre active de celui-ci ;
- la figure 4 montre une vue latérale en coupe longitudinale d'un deuxième mode de réalisation du réacteur selon l'invention, l'installation représentée incluant deux réacteurs positionnés dans une cuve ;
- la figure 5 montre une vue latérale d'un autre mode de réalisation, suivant lequel plusieurs réacteurs sont regroupés (deux dans l'exemple représenté) sur des chambres de collecte communes.

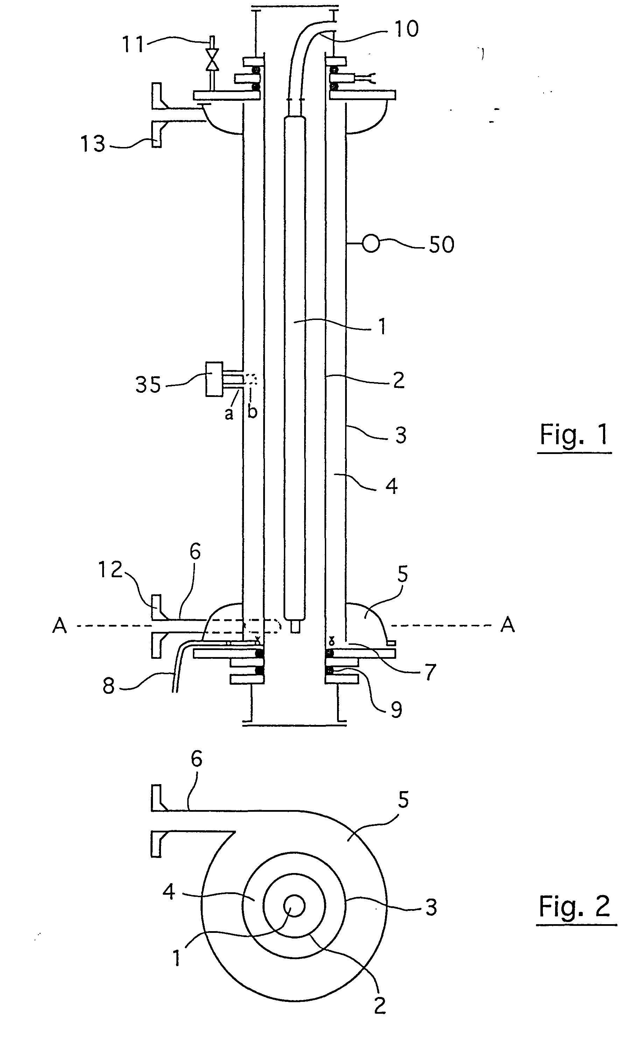

Selon la figure 1, le réacteur d'irradiation UV représenté inclut une lampe UV moyenne pression 1 de puissance nominale 6 kw et installée à l'intérieur d'une gaine de protection réalisée en quartz (matériau transparent aux UV) de diamètre extérieur 140 mm. L'ensemble constitué par la lampe 1 et sa gaine 2 est disposé à l'intérieur d'une virole 3 métallique de diamètre intérieur 200 mm coaxialement à celle-ci. La paroi intérieure de la virole 3 et la paroi extérieure de la gaine 2 délimitent une chambre active 4 à l'intérieur de laquelle un liquide à désinfecter transite. Ce liquide entre dans le réacteur par une entrée 12 située en pied de celui-ci et en sort après avoir été irradié par une sortie 13 prévue en tête du réacteur.

La chambre active 4 est dimensionnée de façon à permettre l'écoulement tubulaire de ce liquide selon une lame d'une épaisseur de 30 mm. On notera qu'en pratique cette épaisseur sera dans d'autres modes de réalisation généralement de l'ordre de 1 à 10 cm. Par ailleurs, la longueur d'irradiation autorisée par le réacteur représenté est de 1 m, tandis que son volume à nettoyer est d'environ 30 litres.

Dans le cas représenté, la virole 3 est réalisée en acier inoxydable 316L polie intérieurement au grade 150.

Un système d'étanchéité par joints toriques 9 isole du milieu liquide l'intérieur de la gaine quartz 2 et l'alimentation électrique de la lampe 1.

Dans la partie inférieure du réacteur représenté une chambre d'injection 5 du liquide à traiter est prévue. Cette chambre 5 est pourvue, comme on peut le voir sur la figure 2 d'une tubulure tangentielle 6 par laquelle le liquide à traiter pénètre dans le réacteur. Cette tubulure 6 assure un mouvement rotatif du liquide dans la chambre d'injection 5 qui minimise la perte de charge d'entrée et améliore la distribution hydraulique du fluide à traiter.

Le liquide sort de la chambre d'injection 5 pour rentrer dans la chambre active 4 illuminée par le rayonnement UV, en passant par une fente circulaire 7 formant une zone de rétreint assurant une perte de charge de l'ordre de 5 à 10 fois supérieure à la perte de charge d'entrée par la tubulure d'injection 6, garantissant ainsi une distribution homogène du fluide à traiter dans la zone d'irradiation UV, et donc une bonne homogénéité des temps de séjour de chaque particule entrante.

Une disposition similaire est ménagée dans la partie supérieure du réacteur pour améliorer encore l'homogénéité des temps de séjour de chaque particule du fluide à traiter dans la zone d'irradiation.

Par ailleurs, le réacteur décrit est également pourvu dans sa partie inférieure de moyens 8 permettant d'injecter de l'air sous pression pour améliorer la turbulence lors du lavage du réacteur comme il sera explicité ci-après. Une purge 11 est prévue dans la partie supérieure du réacteur de façon à évacuer l'air ayant transité dans celui-ci. Il est à noter que l'invention couvre également la possibilité d'injecter de l'ozone, de l'eau oxygénée (ou tout autre réactif susceptible de compléter l'action désinfectante et/ou détoxifiante des UV sur le liquide à traiter), par l'intermédiaire de ces moyens d'injection 8 prévus en pied de réacteur, en profitant de l'amélioration de l'activité photocatalytique permise par la réflexion des UV.

Une tubulure sur le corps du réacteur permet la connexion d'un capteur UV 35 permettant de mesurer le coefficient de transmission UV de la lame de liquide transitant dans la chambre active 4 et/ou le degré d'encrassement du réacteur.

Un capteur de température 50 est également prévu et permet de se protéger contre un fonctionnement de l'émetteur UV sans présence de liquide à traiter, ce qui occasionnerait des dégâts.

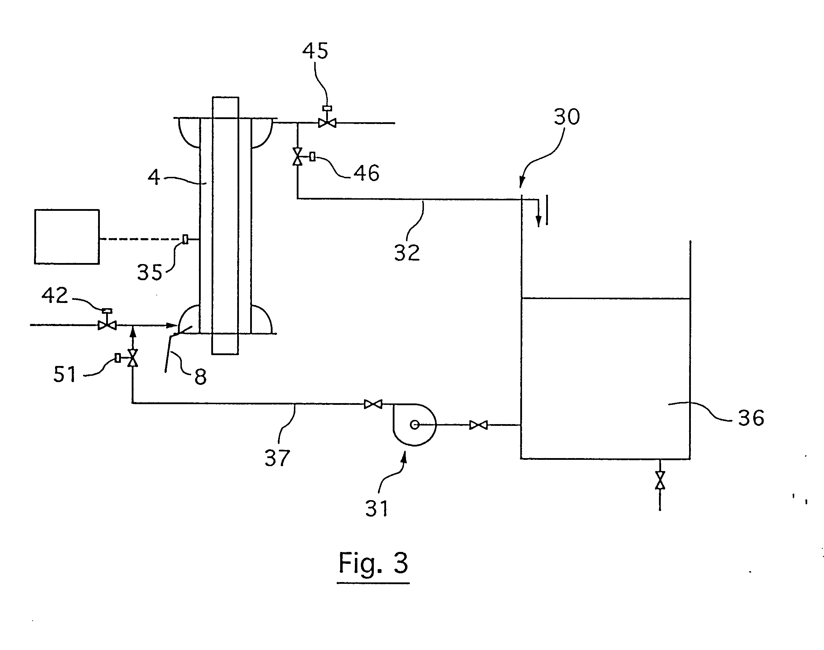

Comme on peut le voir sur la figure 3 le réacteur est relié à des moyens de lavage 30 de la chambre 4 du réacteur permettant de désencrasser celle-ci. Ces moyens de lavage 30 sont reliés au capteur 35 prévu sur le réacteur et sont mis en route dès que la transmission UV détectée par ce capteur passe en deçà d'un seuil prédéterminé. Ils incluent un réservoir de liquide de lavage 36 ainsi qu'une canalisation 37 et une pompe 31 permettant d'envoyer le liquide de lavage contenu dans ledit réservoir à l'intérieur de la chambre 4 du réacteur. Ces moyens de lavage incluent également une canalisation 32 permettant de recycler le liquide de lavage récupéré en tête de réacteur dans le réservoir 36.

En fonction de la nature et du pouvoir encrassant du liquide traité, le réservoir 36 pourra être chargé en divers produits chimiques acide et/ou basique et/ou détergent. On notera que l'on pourra aussi, dans d'autres modes de réalisation de l'invention, choisir de déclencher le lavage selon une grille horaire établie ou encore manuellement à intervalles plus ou moins réguliers. On notera également que l'on pourra prévoir de chauffer le liquide de nettoyage utilisé par exemple à l'aide d'une épingle électrique de chauffage maintenant le liquide de nettoyage dans son réservoir à une température comprise entre 20° et 60° C.

Selon le type de fluide à traiter deux types de lavage pourront d'une manière générale être prévus séparément ou en combinaison :
- lavage à l'aide d'une solution aqueuse acide (acide phosphorique ou acide citrique dilués par exemple) particulièrement pour l'élimination des dépôts minéraux sur les parois chaudes du réacteur ;
- lavage à l'aide d'une solution aqueuse à la soude et/ou aux détergents, particulièrement pour l'élimination des dépôts organiques.

L'efficacité d'un tel lavage est grandement améliorée par les dispositions suivantes, propres à l'invention :
- le poli miroir grain donné à la face intérieure de la paroi du réacteur UV.
- les fortes turbulences engendrées par les vitesses considérables permises par la faible section de passage du réacteur, à savoir 3 cm dans le cadre du présent exemple.
- le très faible volume intérieur du réacteur (de l'ordre de 30 litres pour un réacteur de longueur 1 mètre, puissance 6 kw, sur eau usée) qui permet la recirculation des réactifs avec un minimum de pertes à chaque opération de lavage. Les lavages peuvent ainsi être multipliés sans conséquence importantes en coût de réactif, mais en minimisant d'autant les coefficients d'encrassement à prendre en compte.

Comme précisé ci-dessus l'efficacité du lavage est aussi améliorée par injection d'air en pied de réacteur grâce aux moyens d'injection 8

On notera que de tels moyens de lavage permettent de s'affranchir totalement de la nécessité d'un lavage mécanique par racleurs tels ceux décrits par exemple dans les brevets W0 92/22502 ou EP O 467465A1, d'efficacité limitée et de fiabilité peu sure.

Selon l'invention le lavage du réacteur pourra être effectué alors que l'émetteur (UV) est en fonctionnement, limitant ainsi le vieillissement des émetteurs UV qui est accéléré lors de cycles marche-arrêt répétés.

Lorsque le lavage est décidé la vanne d'entrée 42 est fermée et la vanne d'entrée de réactif de lavage 51 ouverte. Après évacuation du contenu initial du réacteur en liquide à traiter, la vanne de sortie 45 est fermée et, simultanément, la vanne de recyclage 46 ouverte. Après lavage en circuit fermé durant le temps prévu, la vanne 51 est fermée à nouveau, la vanne 42 d'entrée de liquide à traiter ouverte et, avec un décalage programmé, la vanne de recyclage 46 est fermée et la vanne de sortie de liquide traité 45 ouverte.

Suivant un autre mode opératoire destiné à minimiser la consommation de réactif de lavage, l'émetteur UV sera isolé et éteint avant lavage, et le réacteur vidangé de son contenu en eau à traiter avant introduction du liquide de nettoyage.

Le réacteur selon les figures 1 à 3 a été mis en oeuvre avec une eau usée présentant un transmissibilité UV de 40 à 46 % à un débit de 40 m3/ h et avec un lavage à l'acide phosphorique à 5 % toutes les 24 heures. Les résultats suivants sur l'abattement de bactéries coliformes ont été obtenus :

| | |
|---|---|
| coliformes totaux entrée | 1 à 7.107/100 ml |
| coliformes thermo tolérants entrée | 1 à 6.106/100 ml |
| coliformes totaux sortie (à 5.91 kw) | < 408 par 100 ml |
| coliformes thermos-tolérants sortie (à 5.91 kw) | < 52 par 100 ml. |

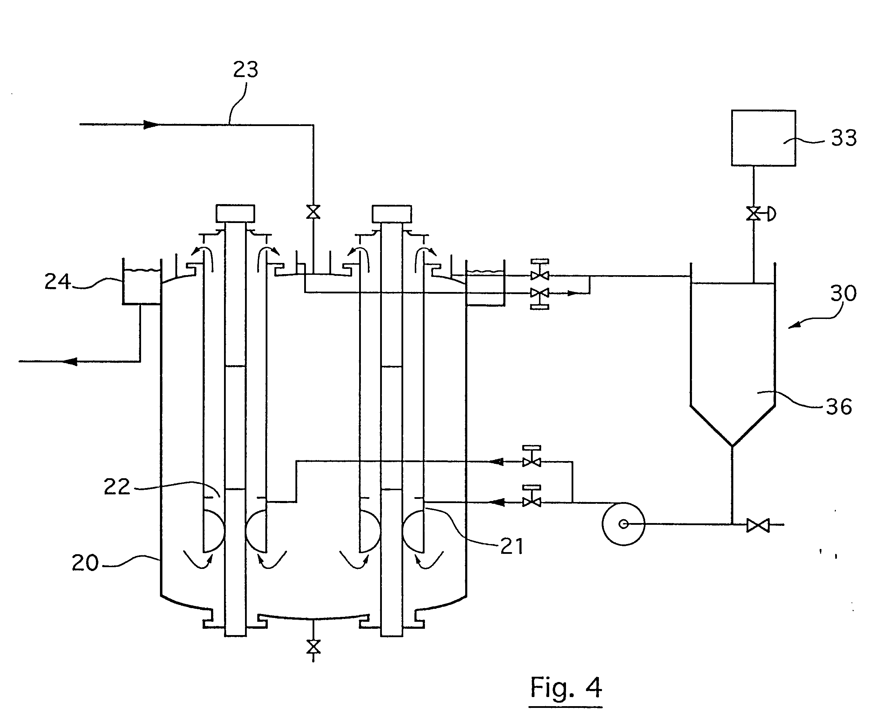

En référence à la figure 4, un autre mode de réalisation de réacteur selon l'invention est représenté. Selon cette figure deux réacteurs sont installés en position verticale dans une cuve 20. Dans ce mode de réalisation la perte de charge en entrée du réacteur est assurée par la perte de charge symétrique propre de la partie active du réacteur (passage rétréci 22 entre la virole tubulaire 3 du réacteur et la gaine tubulaire transparente aux UV protégeant l'émetteur), largement supérieure à la perte de charge dissymétrique engendrée par les changements de direction des flux hydraulique dans le volume tampon situé sous et autour des réacteurs. Dans ce mode de réalisation, le liquide à traiter arrive dans la cuve 20 par une canalisation 23 et le liquide traité sort des réacteurs par des gouttières périphériques 24 prévues à la tête de ces réacteurs.

L'installation comprend par ailleurs des moyens de lavage 30 analogues à ceux du mode de réalisation montré à la figure 3 avec en plus des moyens de réajustement 33 du titre de la solution de nettoyage contenue dans le réservoir 36.

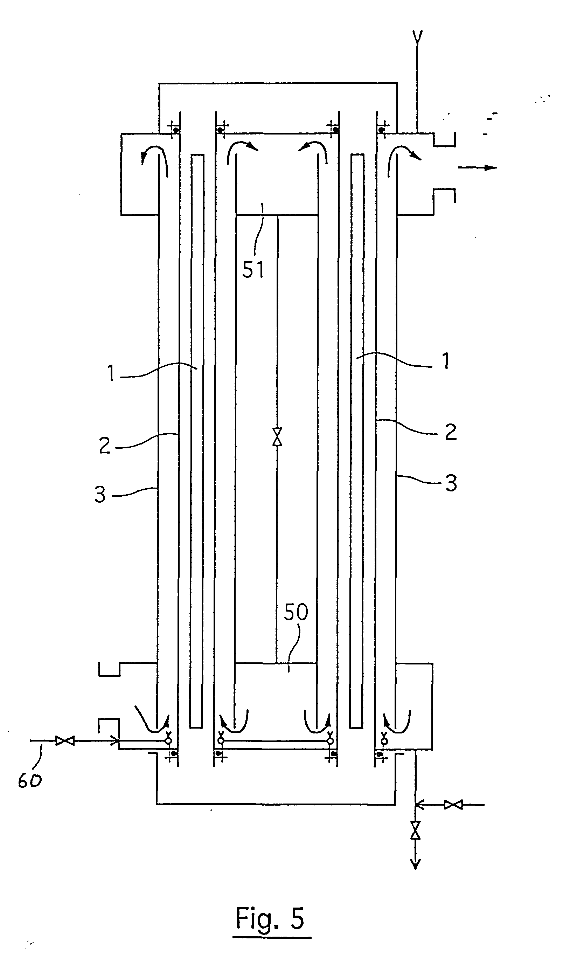

En référence à la figure 5, un autre mode de réalisation de réacteur suivant l'invention est présenté.

Dans ce mode de réalisation, des chambres de répartition hydraulique 50 et 51 communes sont réalisées en alimentation et la sortie d'une série de réacteurs (ici, 2 réacteurs sont représentés) comportant chacun un émetteur UV 1, une gaine de protection transparente aux UV 2, et une virole polie intérieurement 3.L'installation comprend par ailleurs des moyens de lavage 60.

Les modes de réalisation de l'invention ici représentés n'ont pas pour objet de réduire la portée de celle-ci. En particulier il est à noter que le réacteur UV pourra être disposé dans une position quelconque (horizontale, verticale ou incliné) sauf lorsqu'on désirera y adjoindre la possibilité d'une injection d'air comprimé ou de gaz réactifs, qui demande préférentiellement une position verticale (entrée du fluide à traiter par l'extrémité inférieure de réacteur). Le nombre de réacteur et de réactifs de lavage pourra aussi être augmenté de manière évidente pour l'homme de l'art sans modification du principe de fonctionnement.On notera également que plusieurs réacteurs pourront être mis en parallèle ou en série, en utilisant n'importe lequel des moyens de répartition hydraulique bien connus de l'homme de l'art.

## Revendications

1. Réacteur d'irradiation UV pour la désinfection de liquides comportant au moins un émetteur UV haute ou moyenne pression (1) disposé coaxialement à l'intérieur d'une gaine de protection (2) transparente aux UV, l'ensemble constitué par ledit émetteur et ladite gaine étant installé coaxialement à l'intérieur d'une virole tubulaire (3) dont la face intérieure de ladite virole (3) réfléchit au moins 30 % des UV 250 à 260 nm incidents provenant dudit émetteur (1), le liquide à traiter transitant selon un écoulement tubulaire dans une chambre active (4) délimitée par ladite face intérieure de ladite virole (3) et la face extérieure de ladite gaine (2), ledit réacteur étant
**caractérisé en ce qu'**il est relié à des moyens de lavage (30) de ladite chambre active (4) incluant des moyens permettant de faire circuler un liquide de nettoyage dans ladite chambre active (4) et des moyens d'injection (8) d'air prévus en amont de ladite chambre active (5) permettant d'améliorer la turbulence dudit liquide de nettoyage lors du lavage,
en ce qu'il présente des moyens permettant de faire subir au liquide à traiter entrant dans ladite chambre active (4) une perte de charge symétrique au moins trois fois supérieure à la perte de charge dissymétrique observée par le liquide à traiter avant son entrée dans ledit réacteur,
et en ce qu'il comprend une chambre d'injection (5) prévue coaxiale à une extrémité de ladite virole (3) et au moins une tubulure (6) permettant l'introduction du liquide à traiter dans ledit réacteur via ladite chambre d'injection (5) ladite chambre d'injection communiquant avec ladite chambre active (4) par une fente circulaire (7).

2. Réacteur selon la revendication 1 **caractérisé en ce que** lesdits moyens de lavage (30) incluent des moyens permettant de recycler (32) ledit liquide de nettoyage.

3. Réacteur selon l'une des revendications 1 ou 2 **caractérisé en ce que** lesdits moyens de lavage (30) incluent des moyens de réajustement (33) du titre dudit liquide de nettoyage.

4. Réacteur selon l'une des revendications 1 à 3 **caractérisé en ce qu'**il inclut des moyens d'activation automatique (34) desdits moyens de lavage (30).

5. Réacteur selon la revendication 4 **caractérisé en ce que** lesdits moyens d'activation comprennent un capteur UV (35) prévu au niveau de ladite virole (3) du réacteur.

6. Réacteur selon l'une des revendications 1 à 5 **caractérisé en ce qu'**il comprend des moyens de chauffage dudit liquide de nettoyage.

7. Réacteur selon la revendication 1 à 6 **caractérisé en ce que** ladite virole (3) est en acier inoxydable, ladite face intérieure ayant subi un poli miroir de grain 100 à 350.

8. Réacteur selon l'une des revendications 1 à 7 **caractérisé en ce que** ladite face intérieure de ladite virole (3) est constituée par une couche métallique obtenue par dépôt électrolytique.

9. Réacteur selon l'une des revendications 1 à 8 **caractérisé en ce qu'**il est dimensionné de façon à inculquer, au débit considéré de liquide à traiter, un nombre de Reynolds supérieur à 6000 dans l'écoulement tubulaire interne du réacteur.

10. Réacteur selon la revendication 1 à 9 **caractérisé en ce qu'**il comprend des moyens d'injection d'au moins un réactif apte à compléter la désinfection et/ou à détoxifier ledit liquide, lesdits moyens d'injection étant prévus en amont de ladite chambre active.

11. Réacteur selon l'une des revendications 1 à 10 **caractérisé en ce qu'**il comprend des moyens de régulation de la puissance dudit émetteur en fonction d'un ou plusieurs paramètres choisis dans le groupe constitué par le débit de liquide traité, le transmission UV du liquide traité, la durée écoulée depuis le dernier lavage.

12. Réacteur d'irradiation UV pour la désinfection de liquides comportant au moins un émetteur UV haute ou moyenne pression (1) disposé coaxialement à l'intérieur d'une gaine de protection (2) transparente aux UV, l'ensemble constitué par ledit émetteur et ladite gaine étant installé coaxialement à l'intérieur d'une virole tubulaire (3) dont la face intérieure de ladite virole (3) réfléchit au moins 30 % des UV 250 à 260 nm incidents provenant dudit émetteur (1), le liquide à traiter transitant selon un écoulement tubulaire dans une chambre active (4) délimitée par ladite face intérieure de ladite virole (3) et la face extérieure de ladite gaine (2), ledit réacteur étant destiné à être placé dans une cuve de plus grandes dimensions limitant l'effet de l'arrivée dissymétrique du liquide à traiter
**caractérisé en ce qu'**il est relié à des moyens de lavage (30) de ladite chambre active (4) incluant des moyens permettant de faire circuler un liquide de nettoyage dans ladite chambre active (4) et des moyens d'injection (8) d'air prévus en amont de ladite chambre active (5) permettant d'améliorer la turbulence dudit liquide de nettoyage lors du lavage,
en ce qu'il présente des moyens permettant de faire subir au liquide à traiter entrant dans ladite chambre active (4) une perte de charge symétrique au moins trois fois supérieure à la perte de charge dissymétrique observée par le liquide à traiter avant son entrée dans ledit réacteur, et
en ce qu'il présente dans sa partie inférieure un espace annulaire (21) compris entre ladite virole tubulaire (3) et ladite gaine de protection (2) et séparé de ladite chambre active (4) par une passage rétréci (22).

13. Procédé d'utilisation d'un réacteur selon l'une des revendications 1 à 12 **caractérisé en ce qu'**il consiste à faire fonctionner ledit émetteur (1) lorsque les moyens de lavage (30) sont mis en oeuvre.

14. Procédé de régulation d'un système de plusieurs réacteurs UV comportant chacun au moins un émetteur UV haute ou moyenne pression selon l'une des revendications 1 à 12 **caractérisé en ce que** la puissance nécessaire au traitement d'un débit global donné de liquide à traiter est obtenue en répartissant la puissance totale nécessaire sur le nombre d'émetteurs disponibles, plutôt qu'en utilisant quelques émetteurs à leur puissance maximale.

## Claims

1. UV irradiation reactor for disinfecting liquids, comprising at least one high-pressure or medium-pressure UV emitter (1) placed coaxially inside a UV-transparent protective jacket (2), the assembly consisting of the said emitter and the said jacket being installed coaxially inside a tubular envelope (3), the internal face of the said envelope (3) of which reflects at least 30% of the incident 250 to 260 nm UV radiation coming from the said emitter (1), the liquid to be treated passing as a tubular flow through an active chamber (4) bounded by the said internal face of the said envelope (3) and the external face of the said jacket (2), the said reactor being
**characterized in that** it is connected to means (30) for scrubbing the said active chamber (4), including means which make it possible for a cleaning liquid to flow through the said active chamber (4) and air injection means (8) provided upstream of the said active chamber (4) which make it possible to improve the turbulence of the said cleaning liquid during scrubbing, in that it has means making it possible for the liquid to be treated entering the said active chamber (4) to be subjected to a symmetrical pressure drop at least three times greater than the unsymmetrical pressure drop seen by the liquid to be treated before its entry into the said reactor and in that it comprises an injection chamber (5), intended to be coaxial, at one end of the said envelope (3) and at least one port (6) for introducing the liquid to be treated into the said reactor via the said injection chamber (5), the said injection chamber communicating with the said active chamber (4) via a circular slot (7).

2. Reactor according to Claim 1, **characterized in that** the said scrubbing means (30) include means (32) allowing the said cleaning liquid to be recycled.

3. Reactor according to either of Claims 1 and 2,
**characterized in that** the said scrubbing means (30) include means (33) for readjusting the titre of the said cleaning liquid.

4. Reactor according to one of Claims 1 to 3,
**characterized in that** it includes activation means (34) for automatically activating the said scrubbing means (30).

5. Reactor according to Claim 4, **characterized in that** the said activation means comprise a UV sensor (35) provided in the said envelope (3) of the reactor.

6. Reactor according to one of Claims 1 to 5,
**characterized in that** it comprises means for heating the said cleaning liquid.

7. Reactor according to Claims 1 to 6,
**characterized in that** the said envelope (3) is made of stainless steel, the said internal face having been subjected to a 100 to 350 grit mirror polish.

8. Reactor according to one of Claims 1 to 7,
**characterized in that** the said internal face of the said envelope (3) consists of a metal layer obtained by electroplating.

9. Reactor according to one of Claims 1 to 8,
**characterized in that** it is dimensioned so as to force, on the flow in question of liquid to be treated, a Reynolds number greater than 6 000 in the internal tubular flow through the reactor.

10. Reactor according to Claims 1 to 9,
**characterized in that** it comprises means for injecting at least one reactant capable of completing the disinfection and/or of detoxifying the said liquid, the said injection means being provided upstream of the said active chamber.

11. Reactor according to one of Claims 1 to 10,
**characterized in that** it comprises means for regulating the power of the said emitter according to one or more parameters chosen from the group consisting of the flow rate of treated liquid, the UV transmission of the treated liquid and the time since the previous scrubbing.

12. UV irradiation reactor for disinfecting liquids, comprising at least one high-pressure or medium-pressure UV emitter (1) placed coaxially inside a UV-transparent protective jacket (2), the assembly consisting of the said emitter and the said jacket being installed coaxially inside a tubular envelope (3), the internal face of the said envelope (3) of which reflects at least 30% of the incident 250 to 260 nm UV radiation emanating from the said emitter (1), the liquid to be treated passing as a tubular flow through an active chamber (4) bounded by the said internal face of the said envelope (3) and the external face of the said jacket (2), the said reactor being intended to be placed in a tank of larger dimensions, limiting the effect of the unsymmetrical inflow of the liquid to be treated, **characterized in that** it is connected to means (30) for scrubbing the said active chamber (4), which includes means allowing a cleaning liquid to flow through the said active chamber (4) and
air injection means (8) provided upstream of the said active chamber (5) which makes it possible to improve the turbulence of the said cleaning liquid during scrubbing, in that it has means allowing the liquid to be treated entering the said active chamber (4) to be subjected to a symmetrical pressure drop at least three times greater than the unsymmetrical pressure drop seen by the liquid to be treated before its entry into the said reactor and in that it has, in its lower part, an annular space (21) lying between the said tubular envelope (3) and the said protective jacket (2) and separated from the said active chamber (4) by a restricted passage (22).

13. Method of using a reactor according to one of Claims 1 to 12, **characterized in that** it consists in making the said emitter (1) operate when the scrubbing means (30) are being used.

14. Method of regulating a system of several UV reactors, each having at least one high-pressure or medium-pressure UV emitter according to one of Claims 1 to 12, **characterized in that** the power needed for the treatment of a given overall flow of liquid to be treated is obtained by distributing the necessary total power over the number of emitters available, rather than using several emitters at their maximum power.

## Patentansprüche

1. UV-Bestrahlungsreaktor zum Desinfizieren von Flüssigkeiten, der mindestens einen Mittel- oder Hochdruck-UV-Sender (1) umfaßt, welcher koaxial innerhalb einer für UV-Licht durchlässigen Schutzhülle (2) eingesetzt ist, wobei die aus dem Sender und der Hülle gebildete Gruppe koaxial innerhalb einer rohrförmigen Ummantelung (3) angebracht ist, wobei die Innenwand dieser Ummantelung (3) mindestens 30 % der aus dem Sender (1) einfallenden 250 bis 260 nm UV-Strahlung reflektiert, und wobei die zu behandelnde Flüssigkeit durch eine rohrförmige Ableitung in eine Aktivkammer (4) fließt, welche durch die Innenwand der Ummantelung (3) und der Außenwand der Schutzhülle (2) gebildet wird;
der Reaktor ist **dadurch gekennzeichnet, daß** er mit Mitteln zum Spülen (30) der Aktivkammer (4) verbunden ist, welche wiederum Mittel umfassen, mit denen eine Reinigungsflüssigkeit in der Aktivkammer (4) in Umlauf gesetzt werden kann sowie Mittel zum Einspritzen von Luft (8), die oberhalb der Aktivkammer (5) angebracht sind und eine Verbesserung der Wirbelströmung der Reinigungsflüssigkeit während des Spülvorgangs gewährleisten, und daß er über Mittel verfügt, durch welche die zu behandelnde Flüssigkeit, die in die Aktivkammer (4) eintritt, einen mindestens dreimal stärkeren symmetrischen Lastenverlust erleidet als der unsymmetrische Lastenverlust, welche diese zu behandelnde Flüssigkeit vor ihrem Eintritt in den Reaktor erleidet und,
daß er eine Einspritzkammer (5) umfaßt, die koaxial an einem Ende der Ummantelung (3) angebracht ist sowie mindestens eine Rohrleitung (6), mit der die zu behandelnde Flüssigkeit in den Reaktor über die Einspritzkammer (5) eingeleitet werden kann, wobei diese Einspritzkammer mit der Aktivkammer (4) über einen kreisförmigen Schlitz (7) verbunden ist.

2. Reaktor nach Anspruch 1, **dadurch gekennzeichnet, daß** die Mittel zum Spülen (30) Mittel zum Rezyklieren (32) der Reinigungsflüssigkeit umfassen.

3. Reaktor nach einem der Ansprüche 1 oder 2, **dadurch gekennzeichnet, daß** die Mittel zum Spülen (30) Mittel zur erneuten Titrierungseinstellung (33) der Reinigungsflüssigkeit umfassen.

4. Reaktor nach einem der Ansprüche 1 bis 3, **dadurch gekennzeichnet, daß** er über Mittel zum automatischen Aktivieren (34) der Mittel zum Spülen (30) umfaßt.

5. Reaktor nach Anspruch 4, **dadurch gekennzeichnet, daß** die Aktivierungsmittel einen UV-Fühler (35) umfassen, der auf der Höhe der Ummantelung (3) des Reaktors angebracht ist.

6. Reaktor nach einem der Ansprüche 1 bis 5, **dadurch gekennzeichnet, daß** er über Mittel zum Heizen der Reinigungsflüssigkeit verfügt.

7. Reaktor nach einem der Ansprüche 1 bis 6, **dadurch gekennzeichnet, daß** die Ummantelung (3) aus Edelstahl ist, wobei die Innenwand einen Spiegelschliff mit einem Schleifmittel der Körnung 100 bis 350 erhalten hat.

8. Reaktor nach einem der Ansprüche 1 bis 7, **dadurch gekennzeichnet, daß** die Innenwand der Ummantelung (3) eine elektrolytisch aufgetragene Metallschicht aufweist.

9. Reaktor nach einem der Ansprüche 1 bis 8, **dadurch gekennzeichnet, daß** er so bemessen ist, daß die zu reinigende Flüssigkeit bei dem betrachteten Durchsatz innerhalb des reaktorinneren Ablaufrohrs eine Reynoldszahl von mehr als 6000 aufweist.

10. Reaktor nach einem der Ansprüche 1 bis 9, **dadurch gekennzeichnet, daß** er über Mittel zum Einspritzen mindestens einer Reagenzie verfügt, die in der Lage ist, die zu behandelnde Flüssigkeit zu Ende zu desinfizieren und/oder diese Flüssigkeit zu entgiften, wobei diese Einspritzmittel oberhalb der Aktivkammer angebracht sind.

11. Reaktor nach einem der Ansprüche 1 bis 10, **dadurch gekennzeichnet, daß** er über Mittel zum Regeln der Leistung des Senders als Funktion eines oder mehrerer Parameter verfügt, welche aus der Gruppe gewählt werden, die von dem Durchsatz an behandelter Flüssigkeit, von der UV-Durchlässigkeit dieser Flüssigkeit und von der seit der letzten Spülung vergangenen Zeit gebildet wird.

12. UV-Bestrahlungsreaktor zum Desinfizieren von Flüssigkeiten, der mindestens einen Mittel- oder Hochdruck-UV-Sender (1) umfaßt, welcher koaxial innerhalb einer für UV-Licht durchlässigen Schutzhülle (2) eingesetzt ist, wobei die aus dem Sender und der Hülle gebildete Gruppe koaxial innerhalb einer rohrförmigen Ummantelung (3) angebracht ist, wobei die Innenwand dieser Ummantelung (3) mindestens 30 % der aus dem Sender (1) einfallenden 250 bis 260 nm UV-Strahlung reflektiert, und wobei die zu benandelnde Flüssigkeit durch eine rohrförmige Ableitung in eine Aktivkammer (4) fließt, welche durch die Innenwand der Ummantelung (3) und der Außenwand der Schutzhülle (2) gebildet wird, wobei der Reaktor in eine größere Wanne eingesetzt wird, welche den Einfluß des unsymmetrischen Einlaufs der zu behandelnden Flüssigkeit begrenzen soll,
**dadurch gekennzeichnet, daß** er mit Mitteln zum Spülen (30) der Aktivkammer (4) verbunden ist, welche über Mittel zum Zirkulieren einer Reinigungsflüssigkeit durch die Aktivkammer (4) verfügen sowie über Mittel zum Einspritzen von Luft (8), die oberhalb der Aktivkammer (5) angebracht sind und die Wirbelströmung der Reinigungsflüssigkeit beim Spülvorgang verbessern und,
daß er über Mittel verfügt, durch welche die zu behandelnde Flüssigkeit, die in die Aktivkammer (4) eintritt, einen mindestens dreimal stärkeren symmetrischen Lastenverlust erleidet als der unsymmetrische Lastenverlust, welche diese zu behandelnde Flüssigkeit vor ihrem Eintritt in den Reaktor erleidet und,
daß er am unteren Teil einen Ringraum (21) umfaßt, der sich zwischen der rohrförmigen Ummantelung (3) und der Schutzhülle (2) befindet und von der Aktivkammer (4) durch einen verengten Durchgang (22) getrennt ist.

13. Verfahren zur Verwendung eines Reaktors nach einem der Ansprüche 1 bis 12, **dadurch gekennzeichnet, daß** der Sender (1) dann eingeschaltet wird, wenn die Mittel zum Spülen (30) verwendet werden.

14. Verfahren zum Regeln eines Systems mehrerer UV-Reaktoren, die jeweils mindestens einen Mittel- oder Hochdruck-UV-Sender nach einem der Ansprüche 1 bis 12 umfassen,
**dadurch gekennzeichnet, daß** die zum Behandeln einer gegebenen Gesamtmenge von Flüssigkeit benötigten Leistung durch Verteilen der insgesamt benötigten Leistung über die Zahl der verfügbaren Sender verteilt wird, an Stelle des Einsatzes einiger Sender bei ihrer Höchstleistung.
